# DEMANDE DE BREVET EUROPEEN

(11) **EP 1 354 568 A2**
(43) Date de publication de la demande: **22.10.2003**
(21) Numéro de dépôt: 03290885.7
(22) Date de dépôt: 09.04.2003
(51) Int. Cl.: A61F 2/00

(54) **Dispositif de traitement de l'incontinence urinaire**

(30) Priorité: 17.04.2002 FR 0204796
(71) Demandeur: Centre Hospitalier Universitaire de Rouen, 76000 Rouen (FR)
(72) Inventeur: Grise, Philippe, 76420 Bihorel (FR)
(74) Mandataire: Santarelli

(57) **Abrégé**

Constitution d'une fronde sous-urétrale raccordée à deux ballons situés de part et d'autre de l'urètre.

Le dispositif comprend deux sous-ensembles (12a, 12b) comprenant chacun un ballon (13) gonflable et une bandelette (16) dont une extrémité est fixée à la surface du ballon. Les deux bandelettes (16) sont raccordées pour former une fronde sous-urétrale.

## Description

L'invention concerne un dispositif de traitement de l'incontinence urinaire, particulièrement l'incontinence urinaire d'effort (toux, marche, etc.) chez la femme. Le dispositif selon l'invention peut être mis en place par un nouveau procédé chirurgical peu traumatisant.

L'incontinence urinaire chez la femme peut résulter d'un défaut de soutien de la région cervico-urétrale, éventuellement associé à une insuffisance du sphincter urétral. Plusieurs procédés chirurgicaux ont été proposés pour supprimer cette incontinence. Parmi les plus efficaces, on sait qu'un renforcement sous-urétral au moyen d'un cordon permettant de relever l'urètre peut être mis en place en pratiquant une incision vaginale réduite. Ce cordon appelé fronde est raccordé à une aiguille que le chirurgien engage dans l'aponévrose pelvienne. Ladite fronde est mise en place de part et d'autre de l'urètre, sous la vessie et le chirurgien fait ressortir l'aiguille à travers la partie basse de la paroi antérieure de l'abdomen.

On note cependant deux inconvénients. Le premier est un risque de perforation de la vessie pendant l'opération. Le second, post-opératoire, est le risque d'un excès de tension de ladite fronde, entraînant une certaine difficulté à uriner. En effet, la tension est difficile à régler pendant l'opération et n'est pas ajustable après celle-ci. Dans certains cas, une seconde intervention pour "relâcher" ladite fronde est nécessaire.

Une autre technique consiste à introduire de petits ballons gonflables de chaque côté de l'urètre. Le ballon comporte une valve et est gonflé in situ. Il est mis en place à l'aide d'un système d'insertion constitué d'une gaine, d'une aiguille et d'un cathéter.

L'invention propose une combinaison des deux techniques permettant de résoudre notamment le problème du réglage de tension de la fronde sous-urétrale. L'idée de base de l'invention est de constituer une fronde sous-urétrale raccordée à deux ballons situés de part et d'autre de l'urètre.

L'invention propose ainsi un dispositif de traitement de l'incontinence urinaire, notamment chez la femme, comprenant une fronde sous-uréthrale suspendue par des attaches à deux points d'amarrage constitués de deux ballons adaptés à être logés de part et d'autres du col de la vessie.

De manière préférée, l'invention propose un dispositif de traitement de l'incontinence urinaire, caractérisé en ce qu'il comprend deux sous-ensembles destinés à être raccordés et en ce que chaque sous-ensemble comprend un ballon gonflable muni d'une valve et une bandelette fixée à la surface dudit ballon, les bandelettes des deux ballons étant destinées à être rattachées l'une à l'autre pour former une fronde sous-urétrale.

Une fois les deux sous-ensembles mis en place et les deux bandelettes assemblées bout à bout, pour constituer une fronde permettant de relever l'urètre, il est facile de régler la tension de ladite fronde en agissant sur le gonflage des deux ballons. Les bandelettes sont par exemple assemblées à l'aide d'un fil chirurgical d'un côté de l'urètre. Les ballons peuvent être gonflés en les remplissant de sérum physiologique.

D'une façon connue, chaque ballon est avantageusement muni d'une valve. La valve peut être directement fixée au ballon. Si on désire se réserver une possibilité de réglage post-opératoire, on peut utiliser, de façon connue en soi, une variante dans laquelle ladite valve est reliée au ballon par un conduit. Ceci permet une mise en place sous-cutanée de la valve et, par conséquent, une intervention post-opératoire pour le réglage éventuel du gonflage dudit ballon.

Le ballon et la bandelette sont réalisés en un matériau biocompatible (biomatériau) approprié. De manière préférée, le ballon est en silicone ; de même la bandelette est avantageusement réalisée en un matériau synthétique souple, de préférence en polypropylène. La bandelette comporte avantageusement un tressage à maillage large ou, en variante, une surface plane comportant des pores, ce qui facilite son intégration cellulaire. Le ballon peut être de forme sensiblement sphérique ou sensiblement ovoïde.

L'invention sera mieux comprise et d'autres avantages de celle-ci apparaîtront mieux à la lumière de la description qui va suivre, donnée uniquement à titre d'exemple et faite en référence aux dessins annexés dans lesquels :
- la figure 1 représente un dispositif formé de deux sous-ensembles, avant assemblage de leurs bandelettes ;
- la figure 2 représente un sous-ensemble selon une variante possible ; et
- la figure 3 est un schéma illustrant l'implantation du dispositif de part et d'autre de l'urètre.

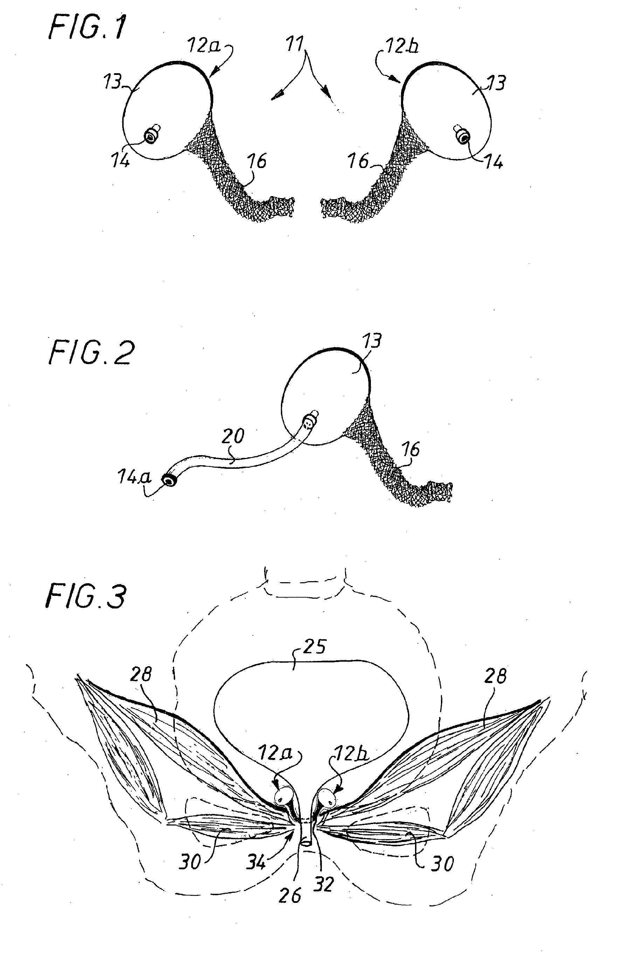

En considérant plus particulièrement la figure 1, on remarque que le dispositif 11, conforme à l'invention selon un mode préféré de réalisation, se compose de deux sous-ensembles 12a, 12b semblables comportant chacun un ballon 13 gonflable muni d'une valve 14 et une bandelette 16 dont une extrémité est fixée à la surface dudit ballon. Les deux bandelettes 16 sont destinées à être rattachées l'une à l'autre pendant l'opération chirurgicale pour former une fronde sous-urétrale. Dans l'exemple de la figure 1, la valve 14 est directement fixée au ballon 13. Ce dernier est, dans l'exemple, en silicone. Il est de forme sensiblement sphérique et, lorsqu'il est rempli, par exemple de liquide physiologique, son diamètre est d'environ 1 cm. Le ballon pourrait être de forme sensiblement ovoïde tout en conservant approximativement les mêmes dimensions. Chaque bandelette 16 peut être en polypropylène ou en tout autre matériau synthétique souple. Elle est de préférence tressée à maillage large pour les raisons indiquées ci-dessus. Dans l'exemple de la figure 2, la valve 14a est reliée au ballon par un conduit 20, pour mise en place sous-cutanée de la valve, selon une technique connue, permettant de réajuster le gonflage du ballon en évitant une nouvelle intervention chirurgicale.

On va maintenant décrire la mise en place du dispositif au cours d'une intervention chirurgicale. Sur la figure 3, on a représenté la vessie 25 prolongée par l'urètre 26. Le but de l'intervention est de "relever" l'urètre par mise en place d'une fronde sous-urétrale ancrée aux deux ballons 12a, 12b placés de part et d'autre de l'urètre, sous la vessie. De part et d'autre de la vessie et de l'urètre, on a représenté l'aponévrose pelvienne 28 et le muscle transverse du périnée 30. Le chirurgien pratique une légère incision vaginale donnant accès à l'arrière de l'urètre. Il pratique ensuite une perforation de l'aponévrose pelvienne au moyen d'un instrument à bout arrondi. Une gaine est enfilée sur cet instrument et un sous-ensemble 12a, 12b (ballon non gonflé) est introduit dans cette gaine et mis en place sous la vessie, sur l'un des côtés de l'urètre. Le ballon est ensuite gonflé. Lorsque les deux sous-ensembles 12a, 12b sont ainsi mis en place de part et d'autre de l'urètre, les bandelettes 16 sont raccordées l'une à l'autre (jonction 32), par du fil chirurgical, ou une agrafe, ou un clip, sur l'un des côtés de l'urètre. Une fronde sous-urétrale 34 est ainsi reconstituée entre les deux ballons. L'incision vaginale est ensuite refermée.

Il est à noter que ce mode opératoire permet d'éviter une perforation de la vessie. Les avantages de l'invention sont les suivants. Pendant l'opération, les ballons sont mis en place de part et d'autre de l'urètre et donc en dessous des faces latérales de la vessie, ce qui élimine les risques de perforation de cette dernière. La tension de la fronde est réglable pendant l'opération car il suffit de suturer l'une des bandelettes à l'autre en donnant à la fronde la longueur voulue en fonction du niveau de tension souhaité. Si nécessaire, la fronde peut être détendue en dégonflant le ou les ballons par simple ponction de liquide physiologique. Par ailleurs, les ballons permettent de renforcer l'aponévrose pelvienne dont la faiblesse éventuelle est une composante du défaut de soutien de la vessie et donc l'une des raisons de l'incontinence. Au repos, les ballons n'exercent pas de pression significative latéro-urétrale. Cependant, lors d'un effort de pression abdomino-pelvienne, la fronde les attire l'un vers l'autre, ce qui renforce l'effet d'occlusion que les ballons peuvent exercer, dans ces conditions, sur l'urètre.

On appréciera que l'ensemble propose ainsi un dispositif de traitement de l'incontinence urinaire chez la femme comprenant une fronde sous uréthrale suspendue par des attaches à deux points d'amarrage constitués de deux ballonnets de forme simple, faciles à loger de part et d'autre du col de la vessie et réalisant à la fois un moyen d'ancrage de la fronde et un moyen de réglage de celle-ci facilement utilisable en per opératoire ou en post opératoire. Le ballon n'est relié à aucun élément fixe, il contribue par sa présence à renforcer un éventuel déficit du fascia pelvien, il exerce lors de la toux ou de l'hyperpression abdominale un effet dynamique qui vise à agir sur la tension de la fronde et sur le col vésical.

Chaque ensemble bandelette-ballonnet est facile à mettre en place.

## Revendications

1. Dispositif de traitement de l'incontinence urinaire, notamment chez la femme, comprenant une fronde sous-uréthrale suspendue par des attaches (10) à deux points d'amarrage constitués de deux ballons (13) adaptés à être logés de part et d'autres du col de la vessie.

2. Dispositif selon la revendication 1, **caractérisé en ce qu'**il comprend deux sous-ensembles (12a, 12b) destinés à être raccordés et **en ce que** chaque sous-ensemble comprend un ballon gonflable (13) muni d'une valve (14) et une bandelette (16) fixée à la surface dudit ballon, les bandelettes des deux ballons étant destinées à être rattachées l'une à l'autre pour former la fronde sous-urétrale.

3. Dispositif selon la revendication 2, **caractérisé en ce qu'**un ballon précité est muni d'une valve (14).

4. Dispositif selon la revendication 3, **caractérisé en ce que** ladite valve (14) est directement fixée audit ballon.

5. Dispositif selon la revendication 3, **caractérisé en ce que** ladite valve (14a) est reliée audit ballon par un conduit (20), pour mise en place sous-cutanée de ladite valve.

6. Dispositif selon l'une quelconque des revendications précédentes, **caractérisé en ce que** la fronde comporte une bandelette (16) en matériau synthétique souple, tressé à maillage large.

7. Dispositif selon la revendication 6, **caractérisé en ce que** ladite bandelette est en polypropylène.

8. Dispositif selon l'une quelconque des revendications précédentes, **caractérisé en ce qu'**un ballon (13) précité est en silicone.

9. Dispositif selon l'une quelconque des revendications précédentes, **caractérisé en ce qu'**un ballon (13) précité est de forme sensiblement sphérique.

10. Dispositif selon l'une quelconque des revendications 1 à 8, **caractérisé en ce qu'**un ballon (13) précité est de forme sensiblement ovoïde.
